# EUROPEAN PATENT SPECIFICATION

(11) **EP 1 424 077 B1**
(45) Date of publication and mention of the grant of the patent: **29.10.2008**
(21) Application number: 02729761.3
(22) Date of filing: 20.05.2002
(51) Int. Cl.: A61K 38/28, A61K 9/12, A61K 9/107

(54) **INSULIN-CONTAINING ORAL SPRAY AND THE PREPARATION METHOD THEREOF**
INSULIN-HALTIGES MUNDSPRAY UND HERSTELLUNGSVERFAHREN DAFÜR
SPRAY BUCCAL CONTENANT DE L'INSULINE ET PROCEDE DE PREPARATION CORRESPONDANT

(30) Priority: 08.08.2001 CN 01128323
(43) Date of publication of application: 02.06.2004
(73) Proprietor: Huazhong University of Science and Technology, Wuhan, Hubei Province 430074 (CN); Hubei Huagong Biochemistry Eng. Co., Ltd., D018 Wuhan, Hubei 430074 (CN)
(72) Inventor: XU, Huibi, Wuhan, Hubei 430074 (CN); HUANG, Kaixun, Wuhan, Hubei 430074 (CN); GAO, Qiuhua, Wuhan, Hubei 430074 (CN); GAO, Zhonghong, Wuhan, Hubei 430074 (CN); YANG, Jilin, Wuhan, Hubei 430074 (CN)
(74) Representative: Vanhala, Jorma Kalevi
(86) International application number: PCT/CN2002/000342
(87) International publication number: WO 2003/013589

(56) References cited:
- WO-A-00/18371
- WO-A-00/21577
- CN-A- 1 264 596
- DE-A1- 19 940 227
- US-A- 5 164 184
- US-B1- 6 191 105
- US-B1- 6 221 378
- PATKI V P ET AL: "PROGRESS MADE IN NON-INVASIVE INSULIN DELIVERY" INDIAN JOURNAL OF PHARMACOLOGY, XX, XX, vol. 28, no. 3, 1996, pages 143-151, XP009081035 ISSN: 0253-7613
- SAYANI A P ET AL: "SYSTEMIC DELIVERY OF PEPTIDES AND PROTEINS ACROSS ABSORPTIVE MUCOSAE" CRITICAL REVIEWS IN THERAPEUTIC DRUG CARRIER SYSTEMS, vol. 13, no. 1/2, 1996, pages 85-184, XP000867488 ISSN: 0743-4863
- XU HUI-BI ET AL: "Hypoglycaemic effect of a novel insulin buccal formulation on rabbits." PHARMACOLOGICAL RESEARCH, vol. 46, no. 5, November 2002 (2002-11), pages 459-467, XP002427259 ISSN: 1043-6618

## Description

### Field of the invention

The present invention provides an insulin-containing formulation and the preparation method thereof, in particularly, an insulin-containing buccal spray for absorption through human buccal mucosa and the preparation method thereof.

### Background of the invention

Insulin is liable to be degraded by the gastric acid and various digestive enzymes in the gastrointestinal tract, thus can not be administrated orally. Generally, Insulin is administrated by injection, and is performed at the time of half an hour before meal to regulate blood glucose. Furthermore, insulin injection would be very inconvenient since patients normally need insulin administration in their whole life. Therefore, pharmaceutical formulations having safe, convenient, and effective property, in particularly non-injection administration would be welcome by patients. The non-injection administration of insulin has become an attractive subject in pharmaceutical field worldwide in recent years. In the last ten years, great advances have been achieved. At present, there are many optional administrating routes of insulin. For example, the administrating route which insulin pump is embedded intraperitoneum is proved to be safe and effective. It has been applied clinically in some countries. However, their prices are too high to be burdened by general patients. Some investigations suggest that the method which insulin is absorbed through bronchial mucosa has attractive applying expectation. However, there are some difficulties to be solved for this method. Some studies focus on oral administration of insulin, i.e. insulin is embedded in liposomes or polymers, thus obtain oral formulation of insulin. The insulin in the formulation is absorbed through mucosal cells of small intestine to achieve the effect of decreasing blood glucose. The low bioavailability and quick clearance from the administrating site are main obstacle to oral administration of insulin. Recently, more and more researches concentrate on administrating route of nasal mucosa, ocular mucosa, pneumal mucosa, and buccal mucosa. Although some advances have been achieved upon ten years studies, the bioavailability of insulin is still low in clinical application since the molecular weight of insulin is too large to be absorbed easily. The key point is to choose suitable absorption promoter having low toxicity and high efficiency to increase bioavailability of insulin.

### Disclosure of the invention

The present invention provides an insulin-containing formulation and the preparation method thereof. The technical problem to be solved by the present invention is to further improve bioavailability of the insulin formulation absorbed through human buccal mucosa and increase stability of the formulation.

The inventor further optimizes the ratio of components of the composition and replace agitating treatment with sonicating treatment, thereby obtain a microemulsion having the average diameter of less than 200nm based on the Chinese patent application 00114318.2.

The present invention provides an insulin buccal spray, which is microemulsion having the average diameter of less than 200nm, comprising 10000u-70000u of insulin, 5-50g of soybean lecithin as absorption promoter, 25-80g of propylene glycol as cosolvent, and balanced with phosphate buffer of pH 6.8-7.8 to 1000ml.

According to the insulin buccal spray of the present invention, the average diameter of the microemulsion is 100-180nm.

The insulin buccal spray of the present invention also comprises borneol-ethanol solution as flavoring agent and phenol as antimicrobial, wherein the amount of borneol is 1.2-10g, the amount of absolute ethanol is 1 - 15ml per 1000ml microemulsion and the amount of phenol is 2g-5g per 1000ml microemulsion.

In a preferred embodiment of the present invention, the microemulsion comprises 15000u-60000u of insulin, 20-50g of soybean lecithin as absorption promoter, 40-80g of propylene glycol as cosolvent, borneol-ethanol solution as flavoring agent prepared by dissolving 1.2-10g of borneol in 1-15ml of absolute ethanol, 2g-5g of phenol as antimicrobial, and balanced with phosphate buffer of pH 6.8-7.8 to 1000ml.

The present invention also provides a method for preparing the insulin buccal spray, comprising: adding 5-50g of soybean lecithin to 25-80g of propylene glycol, then adding 25-60 % by volume of phosphate buffer based on the total amount of phosphate buffer, and sonicating for 0.5-2 hrs, thus obtaining an oil phase of the microemulsion; dissolving 10000u-70000u of insulin in 40-75 % by volume of phosphate buffer based on the total amount of phosphate buffer; adding the insulin solution to the oil phase gradually, and sonicating 2-10 mins.

When the flavoring agent and antimicrobial are added, the method for preparing the insulin buccal spray according to the present invention, comprising: dissolving 2g-5g of phenol in phosphate buffer; adding 20-50g of soybean lecithin to a solution of 40-80g of propylene glycol and borneol-ethanol, then adding 25-60 % by volume of phenol-containing phosphate buffer based on the total amount of phosphate buffer, and sonicating for 0.5-2 hrs, thus obtaining an oil phase of the microemulsion; dissolving 15000u-60000u of insulin in 40-75 % by volume of phenol-containing phosphate buffer based on the total amount of phosphate buffer; adding the insulin solution to the oil phase gradually, and sonicating 2-10 mins.

According to the method for preparing the insulin buccal spray of the invention, the ultrasonic frequency of sonication is 18-25KHz, and the duty ratio of sonication is 30-90 %.

According to the method for preparing the insulin buccal spray of the invention, the temperature in the processing is controlled to a range between 2°C and 70°C.

Since insulin is a polypeptide hormone, its permeability is not good in the case of direct administration through buccal mucosa. Suitable absorption promoter must be chosen to improve bioavailability. The soybean lecithin used in the present invention is a non-toxic, safe, and effective absorption promoter. Furthermore, suitable cosolvent is required due to poor water-solubility of soybean lecithin. The effect of propylene glycol as cosolvent is investigated by the index of the hypoglycemic level in the present invention. The experiments suggest that propylene glycol can improve the effect of absorption of insulin through buccal mucosa that promoted by soybean lecithin. Their best ratio is determined by orthogonal design. The insulin in the mixture of soybean lecithin and propylene glycol form thermodynamics stable system of microemulsion. At the same time, soybean lecithin is used as surfactant and carrier of medicine. The aqueous soluble insulin is embeded in the aqueous solution of lipoidal double layers of soybean lecithin.

The insulin buccal spray of the present invention can include any pharmaceutically acceptable excipents, as long as they do not destroy character of microemulsion and effect of medicament. These pharmaceutically acceptable excipents includes, but not be limited to various diluents, solvents, emulsifiers, preservatives, stabilizers, dissolution aids, flavoring agents, and perfuming agents.

The insulin buccal spray of the present invention has no toxicity. The long term toxicity experiment of rat was carried out by locally administrating the insulin buccal spray in which the dosages were 4.5, 9.0, 18.0 u •kg⁻¹ respectively and physiological saline and carrier were used as control. No obvious abnormality was observed.

The insulin buccal spray prepared by sonicating has significantly improved efficiency and stability as compared with those prepared by previous methods, such as the method described in the Chinese patent application No.00114318.2.
1. Stability. The insulin buccal spray prepared by sonicating according to the invention has significantly smaller diameter of the microemulsion, i.e. nanometer grade, in particularly the average diameter is less than 200nm and distribution of size is between 100nm and 160nm, while the average diameter of previous microemulsion are about 427.2nm. The nanometer grade microemulsion not only favor to absorption through mucosa, but also improve stability of the formulation. The insulin buccal spray prepared by sonicating is translucent at the time of visual inspection. After placed in a freezer of 2-8°C for 1 year, the insulin buccal spray of the invention does not form precipitate. On the contrary, the insulin buccal spray prepared by agitation previously is opaque, and form slight precipitate after placed in a freezer of 2-8°C for 1 year. Therefore, the sonicating treatment is very important to the preparation of microemulsion.
2. Efficiency. The insulin content in vivo in which normal rabbits are used as pharmacological model are determined by enzyme-linked immunoassay. The pharmacokinetic parameter of the insulin buccal spray according to the present invention is calculated. The result suggests that the serum insulin concentration rise rapidly after administrating to normal rabbits through buccal mucosa with the dosage of 1.5 u • kg⁻¹ body weight. The peak time is in the range of between 55 mins and 70 mins and the peak concentration up to 145.2 ± 5.8µ/ml. The serum concentration is decreased to base level after 4-6 hrs. The bioavailability is up to 29.8%. Compared with the control groups without insulin, the groups of administrating insulin through buccal mucosa and those of administrating insulin by subcutaneous injection both show significant difference in the serum insulin concentration of rabbit within 30-120 mins (p<0.05). The groups of administrating insulin through buccal mucosa (1.5u • kg⁻¹) and the control groups of administrating insulin by subcutaneous injection (0.5u • kg⁻¹) have substantively the same peak concentration and peak time, and have no significant difference (p>0.1).

The insulin buccal spray of the present invention can be administrated in single dosage or be divided several dosages, preferably, administrated three times per day (before breakfast, lunch, and supper) or four times per day (before breakfast, lunch, supper, and sleeping). The insulin buccal spray is administrated at the time of one hour before meal. The dosages of the insulin buccal spray of the present invention have no specific limitation and can be administrated based on conventional dosages of insulin. The particular dosages will vary in accordance with individual patients, bioavailability, and severity of conditions, and should be determined by the physicians.

The insulin buccal spray of the present invention is a microemulsion, which has the advantages of large contact area with buccal mucosa, rapid absorption, safety, and excellent hypoglycemic action. The formulation is packaged in a bottle with constant delivery pump, which spray the haze of the formulation to brccal cavity. The insulin is adhered to mucosa of brccal cavity and absorbed rapidly through mucosa to circulation. Thus the rate of absorption is improved significantly. Furthermore, the acidolysis and enzymolysis through gastrointestinal tract and first pass effect of liver can be avoided. The insulin buccal spray of the present invention is a new non-injectable administration mode, which is convenient to patients. The formulation relieves inconvenience and pain of patients suffered diabetes and thus has perfect practicability.

### Examples

### Example 1: 1000ml insulin microemulsion

| | |
|---|---|
| insulin | 40000u |
| soybean lecithin | 25.0g |
| propylene glycol | 75.0g |
| phosphate buffer (pH=7.4) | q.s. to 1000ml |

According to above formulating ratio, 25.0g of soybean lecithin was added to 75.0g of propylene glycol, mixed sufficiently to thin paste. 550ml of phosphate buffer (pH=7.4) was added. Above components were used as carrier of the formulation. The carrier then was sonicated for 1 hr (Condition: the duty ratio of sonication was 50 %, the ultrasonic frequency of sonication was 20KHz, and the temperature was controlled to a range between 40°C and 60°C). 40000u of insulin was dissolved in 350ml of phosphate buffer (pH = 7.4), then slowly added to sonicated carrier solution as prepared above, and continued to sonicate for 5 mins. The average diameter was 160-180 nm determined by laser granularity test instrument,.

The insulin buccal spray of the present invention was prepared in the strictly aseptic condition. The spray prepared was placed in a freezer of 2-8°C for storage.

The insulin buccal spray prepared above has excellent hypoglycemic action. When the diabetic rats induced by streptozotocin were administrated through buccal mucosa with the dosage of 1, 3, 9 u • kg⁻¹, the hypoglycemic ratios were 20.9%, 47.6%, 58.8% respectively. When the diabetic rabbits induced by alloxan were administrated through buccal mucosa with the dosage of 0.5, 1.5, 4.5 u • kg⁻¹, the hypoglycemic ratios were 24.9%, 52.6%, 60.9% respectively.

### Example 2: 1000ml insulin microemulsion

| | |
|---|---|
| insulin | 20000u |
| soybean lecithin | 30.0g |
| propylene glycol | 45.0g |
| borneol | 1.2g |
| absolute ethanol | 3.0ml |
| phenol | 2.0g |
| phosphate buffer (pH=7.4) | q.s. to 1000ml |

According to above formulating ratio, phenol was dissolved in phosphate buffer. 30.0g of soybean lecithin was added to 45.0g of propylene glycol and borneol-ethanol solution. Mixed sufficiently, then 555ml of phenol-containing phosphate buffer (pH=7.4) was added. Above components were used as carrier of the formulation. The carrier then was sonicated for 1.5 hrs (Condition: the duty ratio of sonicating was 60 %, ultrasonic frequency of sonicating was 18KHz, and the temperature was controlled to a range between 50°C and 60°C). 20000u of insulin was dissolved in 345ml of phenol-containing phosphate buffer (pH=7.4), then slowly added to sonicated carrier solution as prepared above, and continued to sonicate for 4 mins. The average diameter was 160-180 nm determined by laser granularity test instrument.

The insulin buccal spray as prepared above was placed in a freezer of 2-8°C for 1 year. No precipitate was formed.

The insulin buccal spray prepared above has excellent hypoglycemic action. When the diabetic rats induced by streptozotocin were administrated through buccal mucosa with dosage of 1, 3, 9 u • kg⁻¹, the hypoglycemic ratios were 21.8%, 47.2%, 56.2% respectively. When the diabetic rabbits induced by alloxan were administrated through buccal mucosa with dosage of 0.5, 1.5, 4.5 u • kg⁻¹, the hypoglycemic ratios were 28.6%, 55.2%, 60.7% respectively.

### Example 3: 1000ml insulin microemulsion

| | |
|---|---|
| insulin | 400000u |
| soybean lecithin | 25.0g |
| propylene glycol | 75.0g |
| borneol | 1.2g |
| absolute ethanol | 4.0ml |
| phenol | 2.0g |
| phosphate buffer (pH = 7.0) | q.s. to 1000ml |

According to above formulating ratio, 25.0g of soybean lecithin was added to 75.0g of propylene glycol and borneol-ethanol solution, mixed sufficiently. 540ml of phenol-containing phosphate buffer (pH=7.0) then was added. Above components were used as carrier of the formulation. The carrier was sonicated for 1 hr (Condition: the duty ratio of sonicating was 50 %, ultrasonic frequency of sonicating was 20KHz, and the temperature was controlled to a range between 40°C and 55 °C). 400000u of insulin was dissolved in 360ml of phenol-containing phosphate buffer (pH=7.0), then slowly added to sonicated carrier solution as prepared above, and continued to sonicate for 5 mins. The average diameter was 130-150 nm determined by laser granularity test instrument.

The insulin buccal spray as prepared above was placed in a freezer of 2-8°C for 1 year. No precipitate was formed.

The insulin buccal spray prepared above has excellent hypoglycemic action. When the diabetic rats induced by streptozotocin were administrated through buccal mucosa with dosage of 1, 3, 9 u • kg⁻¹, the hypoglycemic ratios were 21.3%, 49.8%, 60.2% respectively. When the diabetic rabbits induced by alloxan were administrated through buccal mucosa with dosage of 0.5, 1.5, 4.5 u • kg⁻¹, the hypoglycemic ratios were 29.6%, 55.4%, 62.2% respectively.

### Example 4: 1000ml insulin microemulsion

| | |
|---|---|
| insulin | 60000u |
| soybean lecithin | 35.5g |
| propylene glycol | 80.0g |
| borneol | 1.4g |
| absolute ethanol | 4.0ml |
| phenol | 2.0g |
| phosphate buffer (pH=7.8) | q.s. to 1000ml |

According to above formulating ratio, 35.5g of soybean lecithin was added to 80.0g of propylene glycol and borneol-ethanol solution, and mixed sufficiently. 545ml of phenol-containing phosphate buffer (pH=7.8) then was added. Above components were used as carrier of the formulation. The carrier was sonicated for 2 hrs (Condition: the duty ratio of sonicating was 70 %, ultrasonic frequency of sonicating was 25KHz, and the temperature was controlled to a range between 55 °C and 70°C). 60000u of insulin was dissolved in 360ml of phenol-containing phosphate buffer (pH=7.8), then slowly added to sonicated carrier solution as prepared above, and continued to sonicate for 3 mins. The average diameter was 140-160 nm determined by laser granularity test instrument.

The insulin buccal spray as prepared above was placed in a freezer of 2-8°C for 1 year. No precipitate was formed.

The insulin buccal spray prepared above has excellent hypoglycemic action. When the diabetic rats induced by streptozotocin were administrated through buccal mucosa with dosage of 1, 3, 9 u • kg⁻¹, the hypoglycemic ratios were 18.3%, 35.2%, 44.2% respectively. When the diabetic rabbits induced by alloxan were administrated through buccal mucosa with dosage of 0.5, 1.5, 4.5 u • kg⁻¹, the hypoglycemic ratios were 20.1%, 45.4%, 52.2% respectively.

## Claims

1. An insulin buccal spray, which is microemulsion having the average diameter of less than 200nm, comprising 10000u-70000u of insulin, 5-50g of soybean lecithin as absorption promoter, 25-80g of propylene glycol as cosolvent, and balanced with phosphate buffer of pH 6.8-7.8 to 1000ml.

2. The insulin buccal spray according to Claim 1, which comprises 15000u-60000u of insulin, 20-50g of soybean lecithin as absorption promoter, 40-80g of propylene glycol as cosolvent, and balanced with phosphate buffer of pH 6.8-7.8 to 1000ml.

3. The insulin buccal spray according to Claim 1, wherein the average diameter of the microemulsion is 100-180nm.

4. The insulin buccal spray according to Claim 2, wherein the microemulsion also comprises borneol-ethanol solution as flavoring agent, and the amount of borneol is 1.2-10g, the amount of absolute ethanol is 1-15ml per 1000ml microemulsion.

5. The insulin buccal spray according to Claim 2, wherein the microemulsion also comprises phenol as antimicrobial, and the amount of phenol is 2g-5g per 1000ml microemulsion.

6. A method for preparing the insulin buccal spray according to any one of Claims 1-5, comprising:
adding 5-50g of soybean lecithin to 25-80g of propylene glycol, then adding 25-60 % by volume of phosphate buffer based on the total amount of phosphate buffer, and sonicating for 0.5-2 hrs, thus obtaining an oil phase of the microemulsion;
dissolving 10000u-70000u of insulin in 40-75 % by volume of phosphate buffer based on the total amount of phosphate buffer;
adding the insulin solution to the oil phase gradually, and sonicating 2-10 mins.

7. The method according to Claim 6, comprising:
dissolving 2g-5g of phenol in phosphate buffer;
adding 20-50g of soybean lecithin to a solution of 40-80g of propylene glycol and borneol-ethanol, then adding 25-60 % by volume of phenol-containing phosphate buffer based on the total amount of phosphate buffer, and sonicating for 0.5-2 hrs, thus obtaining an oil phase of the microemulsion;
dissolving 15000u - 60000u of insulin in 40-75 % by volume of phenol-containing phosphate buffer based on the total amount of phosphate buffer;
adding the insulin solution to the oil phase gradually, and sonicating 2-10 mins.

8. The method according to Claim 6, wherein the ultrasonic frequency of sonication is 18-25KHz, and the duty ratio of sonication is 30-90 %.

9. The method according to Claim 7, wherein the ultrasonic frequency of sonication is 18-25KHz, and the duty ratio of sonication is 30-90 %.

10. The method according to Claim 6, wherein the temperature in the processing is controlled to a range between 2°C and 70°C.

11. The method according to Claim 7, wherein the temperature in the processing is controlled to a range between 2°C and 70°C.

## Patentansprüche

1. Insulin-Mundspray, das eine Mikroemulsion mit einem durchschnittlichen Durchmesser von weniger als 200 nm ist, umfassend 10.000u-70.000u Insulin, 5-50 g Sojabohnen-Lecithin als Absorptionsförderer, 25-80 g Propylenglycol als Co-Lösungsmittel, und mit einem Phosphatpuffer von einem pH-Wert von 6,8-7,8 auf 1.000 ml abgeglichen wird.

2. Insulin-Mundspray gemäß Anspruch 1, das 15.000u-60.000u Insulin, 20-50 g Sojabohnen-Lecithin als Absorptionsförderer, 40-80 g Propylenglycol als Co-Lösungsmittel umfaßt und mit einem Phosphatpuffer von einem pH-Wert von 6,8-7,8 auf 1.000 ml abgeglichen wird.

3. Insulin-Mundspray gemäß Anspruch 1, worin der durchschnittliche Durchmesser der Mikroemulsion 100-180 nm beträgt.

4. Insulin-Mundspray gemäß Anspruch 2, worin die Mikroemulsion ferner eine Borneol-Ethanol-Lösung als Aromastoff umfaßt und die Menge an Borneol 1,2-10 g beträgt und die Menge an absolutem Ethanol 1-15 ml pro 1.000 ml Mikroemulsion beträgt.

5. Insulin-Mundspray gemäß Anspruch 2, worin die Mikroemulsion ferner Phenol als antimikrobielles Mittel umfaßt und die Menge an Phenol 2-5 g pro 1.000 ml Mikroemulsion beträgt.

6. Verfahren zur Herstellung des Insulin-Mundsprays gemäß mindestens einem der Ansprüche 1 bis 5, umfassend:
Hinzufügen von 5-50 g Sojabohnen-Lecithin zu 25-80 g Propylenglycol, anschließendes Hinzufügen von 25-60 Vol. %igem Phosphatpuffer, bezogen auf die Gesamtmenge an Phosphatpuffer, und Ultraschallbehandlung für 0,5-2 Std., so daß eine Ölphase einer Mikroemulsion erhalten wird;
Lösen von 10.000u-70.000u Insulin in 40-75 Vol. %igem Phosphatpuffer, bezogen auf die Gesamtmenge an Phosphatpuffer;
schrittweises Hinzufügen der Insulinlösung zu der Ölphase und Ultraschallbehandlung für 2-10 Min.

7. Verfahren gemäß Anspruch 6, umfassend:
Lösen von 2-5 g Phenol in Phosphatpuffer;
Hinzufügen von 20-50 g Sojabohnen-Lecithin zu einer Lösung von 40-80 g Propylenglycol und Borneol-Ethanol, anschließendes Hinzufügen von 25-60 Vol. %igem phenolhaltigem Phosphatpuffer, bezogen auf die Gesamtmenge an Phosphatpuffer, und Ultraschallbehandlung für 0,5-2 Std., so daß eine Ölphase der Mikroemulsion erhalten wird;
Lösen von 15.000u-60.000u Insulin in 40-75 Vol. %igem phenolhaltigem Phosphatpuffer, bezogen auf die Gesamtmenge an Phosphatpuffer;
schrittweises Hinzufügen der Insulinlösung zu der Ölphase und Ultraschallbehandlung für 2-10 Min.

8. Verfahren gemäß Anspruch 6, worin die Ultraschallfrequenz der Ultraschallbehandlung 18-25 kHz beträgt und die relative Einschaltdauer der Ultraschallbehandlung 30-90 % beträgt.

9. Verfahren gemäß Anspruch 7, worin die Ultraschallfrequenz der Ultraschallbehandlung 18-25 kHz beträgt und die relative Einschaltdauer der Ultraschallbehandlung 30-90 % beträgt.

10. Verfahren gemäß Anspruch 6, worin die Temperatur bei der Herstellung in einem Bereich zwischen 2°C und 70°C reguliert wird.

11. Verfahren gemäß Anspruch 7, worin die Temperatur bei der Herstellung in einem Bereich zwischen 2°C und 70°C reguliert wird.

## Revendications

1. Spray buccal à base d'insuline, lequel est une microémulsion possédant un diamètre moyen inférieur à 200 nm, comprenant 10 000 u à 70 000 u d'insuline, 5 g à 50 g de lécithine de soja en tant qu'agent favorisant l'absorption, 25 g à 80 g de propylène glycol en tant que cosolvant et étant équilibré avec un tampon au phosphate à pH 6,8-7,8 jusqu'à 1 000 ml.

2. Spray buccal à base d'insuline selon la revendication 1, comprenant 15 000 u à 60 000 u d'insuline, 20 g à 50 g de lécithine de soja en tant qu'agent favorisant l'absorption, 40 g à 80 g de propylène glycol en tant que cosolvant, et étant équilibré avec un tampon au phosphate à pH 6,8-7,8 jusqu'à 1 000 ml.

3. Spray buccal à base d'insuline selon la revendication 1, dans lequel le diamètre moyen de la microémulsion est de 100 nm à 180 nm.

4. Spray buccal à base d'insuline selon la revendication 2, dans lequel la microémulsion comprend également une solution de bornéol-éthanol en tant qu'agent aromatisant, la quantité de bornéol étant de 1,2 g à 10 g et la quantité d'éthanol absolu étant de 1 ml à 15 ml pour 1 000 ml de microémulsion.

5. Spray buccal à base d'insuline selon la revendication 2, dans lequel la microémulsion comprend également du phénol en tant qu'agent antimicrobien, la quantité de phénol étant de 2 g à 5 g pour 1 000 ml de microémulsion.

6. Procédé de préparation du spray buccal à base d'insuline selon l'une quelconque des revendications 1 à 5, comprenant :
l'ajout de 5 g à 50 g de lécithine de soja à 25 g à 80 g de propylène glycol, puis l'ajout de 25 % à 60 % en volume de tampon au phosphate en se basant sur la quantité totale de tampon au phosphate, et une sonication pendant 0,5 heure à 2 heures, pour ainsi obtenir une phase huileuse de la microémulsion ;
la dissolution de 10 000 u à 70 000 u d'insuline dans 40 % à 75 % en volume de tampon au phosphate en se basant sur la quantité totale de tampon au phosphate ;
l'ajout progressif de la solution d'insuline à la phase huileuse et une sonication pendant 2 minutes à 10 minutes.

7. Procédé selon la revendication 6, comprenant :
la dissolution de 2 g à 5 g de phénol dans un tampon au phosphate ;
l'ajout de 20 g à 50 g de lécithine de soja à 40 g à 80 g de propylène glycol et de bornéol-éthanol, puis l'ajout de 25 % à 60 % en volume de tampon au phosphate contenant du phénol en se basant sur la quantité totale de tampon au phosphate, et une sonication pendant 0,5 heures à 2 heures, pour ainsi obtenir une phase huileuse de la microémulsion ;
la dissolution de 15 000 u à 60 000 u d'insuline dans 40 % à 75 % en volume de tampon au phosphate contenant du phénol en se basant sur la quantité totale de tampon au phosphate ;
l'ajout progressif de la solution d'insuline à la phase huileuse et une sonication pendant 2 minutes à 10 minutes.

8. Procédé selon la revendication 6, dans lequel la fréquence ultrasonore de la sonication est de 18 kHz à 25 kHz et le facteur de marche de la sonication est de 30 % à 90 %.

9. Procédé selon la revendication 7, dans lequel la fréquence ultrasonore de la sonication est de 18 kHz à 25 kHz et le facteur de marche de la sonication est de 30 % à 90 %.

10. Procédé selon la revendication 6, dans lequel la température est maintenue dans une plage comprise entre 2 °C et 70 °C lors du processus.

11. Procédé selon la revendication 7, dans lequel la température est maintenue dans une plage comprise entre 2 °C et 70 °C lors du processus.
